# EUROPEAN PATENT APPLICATION

(11) **EP 3 957 628 A1**
(43) Date of publication of application: **23.02.2022**
(21) Application number: 19850821.0
(22) Date of filing: 27.12.2019
(51) Int. Cl.: C07D 231/26

(54) **PHENYLMETHYLPYRAZOLONE COMPOUND HAVING A NOVEL CRYSTAL FORM, AND METHOD FOR PRODUCING SAME**

(30) Priority: 17.04.2019 UA 201904026
(71) Applicant: Sia Emteko Holding, 1046 Riga (LV)
(72) Inventor: GUMENIUK, Mykola, Kyiv, 03110 (UA)
(74) Representative: Godlewski, Piotr
(86) International application number: PCT/IB2019/061397
(87) International publication number: WO 2020/212748

(57) **Abstract**

This invention relates to a compound of phenylmethylpyrazolone having a crystal form, which corresponds to the parameters determined by the method of powder X-ray diffraction analysis Debye-Scherer, wherein the parameters of the crystal cells correspond to: a (Å) - 10.244 (6) Å, b (Å) - 11.198 (5) Å, c (Å) -15.911 (9) Å, β° - 94.95 (3) °, VÅ3 - 1821 (3) Å3. The angles 2Θ for crystal diffraction patterns correspond to: 4.73°; 5.98°; 9.11°; 9.94°; 11.45°; 14.74°; 15.85°; 17.09°; 21.60°. The interplanar distances of the crystals correspond to: 8.09 Å; 6.42 Å; 4.58 Å; 3.97 Å; 3.12 Å; 2.79 Å; 2.58 Å; 2.37; 1.99 Å. A process of obtaining said compound having a crystal form, involves recrystallization of the obtained substance and contains the following steps: i) dissolving the crystals obtained in the synthesis process in ethanol by heating to 45 ° C under reflux; ii) filtering the resulting solution without cooling through an inert material filter; iii) cooling the solution to 5 ° C to form a precipitate (crystals); iv) washing the crystals obtained with ethyl acetate to remove impurities; v) drying the washed crystals in a rotary evaporator under vacuum.

## Description

The invention relates to pharmaceuticals and medicine, in particular to certain drugs used in cerebral ischemia, which have the ability to inhibit lipid peroxidation, more specifically to drugs that are derivatives of pyrazolone, in particular to phenylmethylpyrazolone.

In the human body, lipid oxidation occurs through a chain reaction [Yamamoto Y, Niki E, Kamiya Y, Shimasaki H. Oxidation of lipids. Oxidation of phosphatidylcholines in homogeneous solution and in water dispersion. Biochim Biophys Acta. 1984;795:332-340; Yamamoto Y, Niki E, Eguchi J, Kamiya Y, Shimasaki H. Oxidation of biological membranes and its inhibition. Free radical chain oxidation of erythrocyte ghost membranes by oxygen. Biochim Biophys Acta. 1985;819:29-36], where a single radical can produce thousands of lipid hydroperoxide molecules, resulting in damage to biological membranes and impaired cell function [Yamamoto Y, Niki E, Kamiya Y, Miki M, Tamai H, Mino M. Free radical chain oxidation and hemolysis of erythrocytes by molecular oxygen and their inhibition by vitamin E. J Nutr Sci Vitaminol (Tokyo) 1986;32:475-479.]. Brain ischemia or ischemia, followed by reperfusion, increases the production of free radicals that damage the membranes of brain cells by oxidizing unsaturated fatty acids of phospholipids into peroxide compounds. This leads to ischemic brain damage, edema, heart attack and neuronopathy. Therefore, neutralizing such oxidation is a very important task. Accordingly, there is a need to create free radical acceptor drugs for the treatment of acute ischemic stroke.

Now it is known compound of phenylmethylpyrazolone (FMP) under the chemical name 1-phenyl-3-methyl-5-pyrazolone, with the molecular formula C10H10N2O. FMP is a compound that neutralizes the above oxidation and can thus be used in the treatment of acute ischemic stroke and in the treatment of lateral amyotrophic sclerosis.

Mentioned FMP is a low molecular weight antioxidant agents that deliberately interact with peroxyl radicals. Due to its amphiphilic properties, FMP absorbs fat and water-soluble peroxyl radicals, transmitting the electron to the radical. Thus, it inhibits lipid oxidation by absorbing water-soluble peroxyl radicals that initiate chain chemical reactions, and fat-soluble peroxyl radicals supporting this chain as well. It was found that the mechanism of action of FMP in the treatment of lateral amyotrophic sclerosis is the absorption of peroxynitrite.

Along with the high efficacy of said compound in relation to these pathologies, there are problems which solution will improve the therapeutic properties of the drug and make it safer.

For example, the specified substance has a low level of solubility, which complicates the use of the drug and the manufacture of its solutions with effective concentration.

It is also known that FMP is unstable upon contact with air or when heated with a solution, during which a substance is destroyed and a large number of harmful impurities are formed.

In addition, in the preparation of drugs with the said compound, a relatively large amount of it settles on the details of the equipment, which leads to losses and the problem of restoring the required concentration of the ready solution.

Mentioned problems partially are solved by adding an agent to increase the solubility in the manufacture of the drug, which significantly prolongs the technological process and requires a complex of additional reagents, as, for example, described in patent CN108324683.

The problem of instability of the specified substance can be overcome by introducing into the composition a stabilizer, as described, for example, in patent CN107823128.

Therefore, said problems are solved by extending the process and introducing new steps into the production method, and also add the constituents to the composition of the finished drug. However, that solves the problem only partially. The active ingredient of the drug requires careful mixing, which is accompanied by a significant loss of the active substance that settles on the walls of the equipment. Or you need to introduce solubilizing agents, which are potentially dangerous.

The introduction into the finished drug the additional components and additional processing increase the stability of the drug during storage in the package, but at the first contact with the air after opening the packed container the process of destruction of the compound is inevitable - the compound itself in the preparation of the drug remains unchanged, has its usual properties, and therefore when it comes into contact with air it begins to collapse.

Thus, **the problems in the production** and use of FMP, which need to be addressed and the actual efforts of the authors of the utility model, is to increase the solubility of the active substance, the stability of the drug during storage and contact with air, as well as finding opportunities to reduce the loss of the active substance in the preparation of the finished medicinal product.

These problems are solved by creating a new polymorphic form of FMP, which acquires new properties due to the proposed features of the crystal structure. A phenylmethylpyrazolone compound having a crystal form which corresponds to the parameters determined by the Debye-Scherer powder X-ray diffraction method, where the parameters of the crystal cells correspond to:

| **Parameter\ unit of measure** | **Result** |
|---|---|
| *a(Å)* | *10.244(6) Å* |
| *b (Å)* | *11.198(5) Å* |
| *c (Å)* | *15.911(9) Å* |
| *β°* | *94.95(3) °* |
| *VÅ³* | *1821(3) Å³* |

The compound wherein the angles 2Θ for the crystal diffraction patterns correspond to: 4.73°; 5.98°; 9.11°; 9.94°; 11.45°; 14.74°; 15.85°; 17.09°; 21.60°. The compound where the interplanar distances of crystals correspond to: 8.09 Å; 6.42 Å; 4.58 Å; 3.97 Å; 3.12 Å; 2.79 Å; 2.58 Å; 2.37; 1.99 Å.

Also, a process for the preparation of a compound having a crystal form is created, involves recrystallization of the obtained substance and comprises the following steps: i) dissolving the crystals obtained in the synthesis them in ethanol by heating to 45 ° C under reflux; ii) filtering the resulting solution without cooling through an inert material filter; iii) cooling the solution to 5 ° C to form a precipitate (crystals); iv) washing the crystals obtained with ethyl acetate to remove impurities; v) drying the washed crystals in a rotary evaporator under vacuum. In the method the stage i) is performed in 65% ethanol, where stage ii) is performed using a filter made of inert material selected from the group: fiberglass, steel, polypropylene; stage iv) is carried out with ethyl acetate solution at room-temperature; stage v) is carried out at 30 °C.

### Detailed description of the utility model

FMP is characterized by keto-enol tautomerism and it has three isomeric structures: keto form, enol form and amine form. Like the neutral molecule, the FMP anion also has three resonance structures. Thus, under physiological conditions (in water at pH 7.4) FMP exists as a mixture of neutral and anionic forms, each containing tautomeric forms and resonance structures. The presence of these properties to some extent provides FMP with antioxidant activity [How is edaravone effective against acute ischemic stroke and amyotrophic lateral sclerosis? Kazutoshi Watanabe, Masahiko Tanaka, Satoshi Yuki, Manabu Hirai, Yorihiro Yamamoto. J Clin Biochem Nutr. 2018 Jan; 62(1): 20-38. Published online 2017 Nov 11].

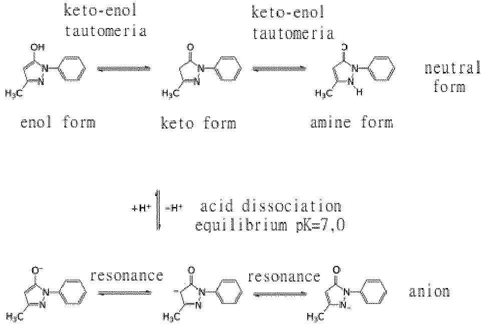

FMP has three isomeric structures: a keto form, an enol form with a hydroxyl group in the 5th position (phenol-like structure), and an amine form with a hydrogen atom on the second atom. Like the neutral molecule, the FMP anion also has three resonance structures.

The keto form is more stable than the enol form, which reacts easier with oxygen and other oxidants. It follows that a change in the keto-enol ratio of the forms of FMP can improve the stability of the compound and its shelf life.

Structural formula of ketone form is:

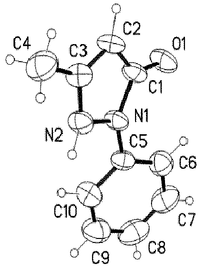

Structural formula of enol form is:

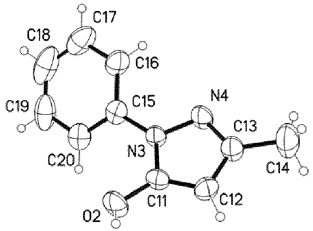

The authors of the utility model studied the properties of FMP and suggested that changing the keto-enol balance of a compound may alter its properties, makes the compound more stable, and extends its shelf life. However, a prerequisite was to keep the therapeutic properties unchanged and increase the safety use. Given that the change in the crystal form of the substance does not change the therapeutic properties, a series of experiments were conducted to change the structure of crystals of FMP molecules.

Further, it is known that the solubility of the compound is influenced by the nature of the crystal lattice, ie, it is possible to improve the soluble properties of the substance by changing the crystal lattice.

Taking in account that the crystal is able to accumulate electrostatic charge on its surface, the problem of settling a relatively large amount of the drug on the walls of the container and on the surface of the parts is due to the electrostatic attraction to the surface of the equipment on which the charge of the opposite sign accumulates. This property may vary depending on the structure of the crystal lattice.

Therefore, a compound having from 60 to 90% of the keto form in the keto-enol ratio of the FMP was obtained experimentally, while the other known polymorphic forms contain about 50% of each form of the FMP. The keto-enol ratio was determined by X-ray diffraction analysis.

The improved stability of the resulting polymorphic form was confirmed by experiments in a climate chamber at 25 ° C and 60% humidity. For 24 months under these conditions, the sample of the substance did not change its key characteristics: the content of the basic substance, the content of impurities, pH.

The new form of FMP provides a solubility of the compound obtained in water of 0.3 mg / ml, while for the known forms, this figure is less than 0.2 mg / ml. The solubility of the obtained samples was checked by dissolving in water with vigorous stirring for 30 minutes at 25 ° C.

Structural analysis of the obtained new crystal form of FMP on the basis of the method of powder X-ray diffraction analysis of Debye-Scherer (method of investigation of the structure of small crystal materials by X-ray diffraction (polycrystal method)) showed its significant differences from the known forms of FMP. The authors of the utility model assume that changes in the crystal lattice were accompanied by changes in the orientation of the molecules in the crystal lattice of the FMP, which in turn led to a change in the ability of the crystal of the compound to accumulate charge.

The following model experiment was performed to test the ability of electrostatic adhesion of the new polymorphic form. The FMP sample was placed in a 316L stainless steel tank, similar to the steel used to make working parts of industrial pharmaceutical equipment. After thorough mixing in the tank, the PMF was released from it and the surface area of the tank covered with the FMP crystals was measured. For the known polymorphic form, this figure was 19.5%, for the new form - 12.8%, which is about one third less.

Therefore, the specified compound was prepared according to a known method of synthesis:

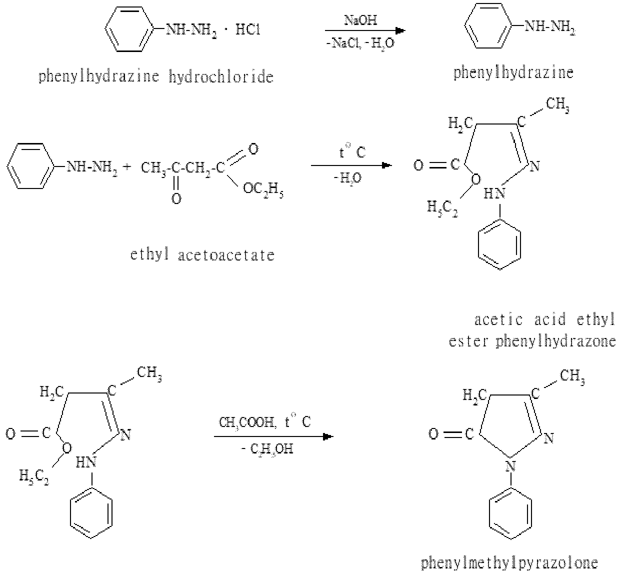

In the following, to change the crystal form and further purification of the substance was carried out recrystallization FMP according to the next general technological scheme:
- Dissolution of the obtained crystals of FMP in 65% ethanol by heating to 45 ° C under reflux.
- Filtration of the resulting solution without cooling through an inert material filter.
- Cooling the solution.
- Washing the obtained FMP crystals at room temperature with ethyl acetate to remove the soluble impurities.
- Drying the washed crystals in a rotary evaporator under vacuum.

Approximate product yield was 60%.

The recrystallization process was carried out in series with a change in temperature and time at the dissolution stage, a change in temperature during cooling and drying of the washed crystals.

The obtained samples were examined by X-ray diffraction analysis.

### Sample # 1

The crystals of FMP obtained in the synthesis process were dissolved in 65% ethanol by heating to 45 ° C under reflux for 30 min.

The resulting solution was filtered without cooling through an inert material filter.

The solution was cooled to 8 ° C.

The obtained FMP crystals were washed at room temperature with ethyl acetate to remove impurities that dissolve well therein for 20 min.

The washed crystals were dried in a rotary evaporator under vacuum at 20 ° C. Monocrystal cell parameters were tested.

Diffraction indices were studied for one of the single crystals and used to determine the structure of the compound by X-ray diffraction analysis. The method of powder X-ray diffraction analysis (Debye-Scherer method) was applied.

### The parameters of cells of crystals according to sample # 1

| **Parameter\crystal** | **1** | **2** | **3** |
|---|---|---|---|
| *a(Å)* | *10.319(6)* | *10.298(4)* | *10.306(4)* |
| *b (Å)* | *11.142(5)* | *11.122(4)* | *11.134(4)* |
| *c (Å)* | *15.870(9)* | *15.833(6)* | *15.831(7)* |
| *β°* | *95.05(3)* | *95.08(1)* | *95.09(1)* |
| *VÅ³* | *1817(3)* | *1806(2)* | *1809(2)* |

| | | | |
|---|---|---|---|
| a (Å) is the length of the crystal cell; b (Å) is the height of the crystal cell; c (Å) is the width of the crystal cell; β° is the symmetry angle of the crystal cell; VÅ3 is the volume of the crystal cell. | | | |

The diffraction patterns were obtained for powder # 1 and the 2θ diffraction angles and the interplanar distances were determined using the Bragg formula.

Angles 2Θ obtained from diffraction patterns for sample # 1 were: 5.15; 6.17; 8.95; 10.00; 11.45; 14.35; 15.44; 16.95; 21.60°.

Plane distances defined for sample # 1 were: 7.91; 6.64; 4.55 Ratio; 4.08; 3.56; 2.84 Hours; 2.65; 2.41; 2.06Å

### Sample # 2

The crystals of FMP obtained in the synthesis process were dissolved in 65% ethanol by heating to 45 ° C under reflux for 20 min.

The resulting solution was filtered without cooling through an inert material filter (glass fiber, steel, polypropylene, etc.).

The solution was cooled to 5 ° C. During cooling, the crystallization (precipitation) of FMP occurs.

The obtained FMP crystals were washed at room temperature with ethyl acetate to remove impurities that dissolve well therein for 20 min.

The washed crystals were dried in a rotary evaporator under vacuum at 30 ° C. Monocrystal cell parameters were tested.

For one of the single crystals, diffraction indices were studied by X-ray diffraction analysis to determine the structure of the compound.

Diffraction indices were studied for one of the single crystals to determine the structure of the compound.

### Cell parameters for sample according to crystals # 2

| **Parameter\ unit of measure** | **Result** |
|---|---|
| *a(Å)* | *10.244(6) Å* |
| *b (Å)* | *11.198(5) Å* |
| *c (Å)* | *15.911(9) Å* |
| *β°* | *94.95(3) °* |
| *VÅ³* | *1821(3) Å³* |

Angles 2Θ for diffraction patterns of crystals of sample # 2 were: 4.73°; 5.98°; 9.11°; 9.94°; 11.45°; 14.74°; 15.85°; 17.09°; 21.60°.

The interplanar distances of the crystals of sample # 2 were: 8.09 Å; 6.42 Å; 4.58 Å; 3.97 Å; 3.12 Å; 2.79 Å; 2.58 Å; 2.37; 1.99 Å.

### Sample # 3

The recrystallization process was carried out according to the scheme for sample # 2, but the washing step was carried out for 30 minutes, and the cooling stage was performed at 10 ° C until precipitation.

This sample did not contain crystals suitable for determining the unit cell parameters, so it was used as a powder to determine the main diffraction angles and interplanar distances.

Angles 2Θ obtained from diffraction patterns for sample # 3 were: 5.24, 6.19, 8.92, 9.98, 11.43, 14.33, 15.46, 16.99, 21.51.

Plane distances were: 7.77; 6.58; 4.57 Ratio; 4.08; 3.57; 2.85; 2.64; 2.41; 1.90°.

### Sample # 4

The recrystallization process was carried out according to the scheme for sample # 2, but the washing step was carried out for 30 min. at a temperature of 40 ° C, and the stage of cooling was performed at a temperature of 10 ° C until precipitation.

The single crystals, suitable for determining the parameters of the unit cell, were found in the sample material.

### The parameters of cells of crystals of sample # 4

| **Parameter\crystal** | **1** | **2** | **3** |
|---|---|---|---|
| *a(Å)* | *10.35(2)* | *10.42(2)* | *10.38(5)* |
| *b (Å)* | *11.03(2)* | *10.96(3)* | *11.13(5)* |
| *c (Å)* | *16.10(4)* | *16.02(3)* | *16.00(7)* |
| *β°* | *94.92(6)* | *95.39(5)* | *95.29(10)* |
| *VÅ³* | *1830(9)* | *1822(9)* | *1859(14)* |

### Sample # 5

The recrystallization process was carried out according to the scheme for sample # 2, but the washing step was carried out for 30 min. at a temperature of 40 ° C, and the stage of cooling was performed at a temperature of 7 ° C to precipitate deposition, the washed crystals were drying at 35 ° C.

The sample did not contain crystals suitable for determining the unit cell parameters, so it was used to determine the main diffraction angles and interplanar distances.

Angles 2Θ obtained from diffraction patterns for sample # 5 were: 5.15, 6.19, 9.00, 9.92, 11.39, 14.24, 15.36, 16.88, 21.40.

Plane distances were: 7.91; 6.58; 4.53; 4.11; 3.58; 2.87; 2.66; 2.42; 1.91.

### Sample # 6

The recrystallization process was carried out according to the scheme for sample # 2, but the washing step was carried out for 35 min. at a temperature of 35 ° C, and the cooling stage - at a temperature of 10 ° C to precipitate deposition, the washed crystals were drying at 35 ° C.

In the sample material were found single crystals, suitable for determining the parameters of the unit cell.

### The parameters of cells of crystals of sample # 6

| **Parameter\sample** | **1** | **2** |
|---|---|---|
| *a (Å)* | *10.33(2)* | *10.35(3)* |
| *b (Å)* | *11.17(3)* | *11.24(3)* |
| *c (Å)* | *15.9039)* | *15.95(3)* |
| *β°* | *95.04(4)* | *94.99(4)* |
| *VÅ³* | *1828(7)* | *1849(8)* |

According to the Cambridge Structural Database (CSD) of the Cambridge Crystallographic Data Center, the substances of the samples correspond to the phenylmethylpyrazolone compound.

Basic bond lengths and valence angles measured for the molecules of the test specimen.

| | |
|---|---|
| C1 O1 1.257(4) | O1 C1 N1 122.1(4) |
| C1 N1 1.377(4) | O1 C1 C2 132.0(3) |
| C1 C2 1.395(5) | N1 C1 C2 105.8(3) |
| C2 C3 1.355(4) | C3 C2 C1 108.5(3) |
| C3 N2 1.341(4) | N2 C3 C2 108.8(4) |
| C3 C4 1.477(5) | N2 C3 C4 119.1(3) |
| C5 C6 1.368(5) | C2 C3 C4 132.1(4) |
| C5 C10 1.382(4) | C6 C5 C10 120.0(4) |
| C5 N1 1.415(4) | C6 C5 N1 120.7(3) |
| C6 C7 1.374(5) | C10 C5 N1 119.4(4) |
| C7 C8 1.366(5) | C5 C6 C7 119.5(4) |
| C8 C9 1.353(6) | C8 C7 C6 120.8(4) |
| C9 C10 1.374(5) | C9 C8 C7 119.5(4) |
| C11 O2 1.334(4) | C8 C9 C10 121.2(4) |
| C11 N3 1.350(4) | C9 C10 C5 119.1(4) |
| C11 C12 1.353(4) | O2 C11 N3 119.2(3) |
| C12 C13 1.389(4) | O2 C11 C12 133.0(3) |
| C13 N4 1.317(4) | N3 C11 C12 107.8(3) |
| C13 C14 1.503(4) | C11 C12 C13 105.6(3) |
| C15 C20 1.378(4) | N4 C13 C12 111.7(3) |
| C15 C16 1.378(4) | N4 C13 C14 119.9(3) . |
| C15 N3 1.414(4) | C12 C13 C14 128.4(3) |
| C16 C17 1.379(5) | C20 C15 C16 120.3(3) |
| C17 C18 1.363(6) | C20 C15 N3 121.0(3) |
| C18 C19 1.371(6) | C16 C15 N3 118.7(3) |
| C19 C20 1.379(5) | C15 C16 C17 119.5(4) |
| N1 N2 1.377(4) | C18 C17 C16 120.7(4) |
| N3 N4 1.381(3) | C17 C18 C19 119.4(4) |
| | C18 C19 C20 121.1(4) |
| | C15 C20 C19 119.0(4) |
| | C1 N1 N2 108.4(3) |
| | C1 N1 C5 129.6(3) |
| | N2 N1 C5 120.9(3) |
| | C3 N2 N1 108.2(3) |
| | C11 N3 N4 110.2(3) |
| | C11 N3 C15 129.9(3) |
| | N4 N3 C15 119.9(2) |
| | C13 N4 N3 104.8(2) |

Sample # 2, which showed the best solubility and electrostatic index was investigated for critical quality indicators compared to the known form of FMP.

### Comparison of critical quality indicators of known and novel polymorphic forms of FMP after 24 months of storage at 25 ° C and 60% relative humidity

| **Indicator** | **Normalizatio n** | **Known form of FMP compound** | | **A new form of FMP compound** | |
|---|---|---|---|---|---|
| | | **0 months** | **24 months** | **0 months** | **24 months** |
| **Description** | White crystalline powder | White crystalline powder | White crystalline powder | White crystalline powder | White crystalline powder |
| **Melting point** | 127-129 C | 127,9 | 128,0 | 128,7 | 128,7 |
| **pH** | 4,5-5,5 | 4,7 | 4,6 | 4,8 | 4,8 |
| **Impurity 1** | Not more than 0,1% | 0,02% | 0,08% | 0,03% | 0,04% |
| **Sum of impurities** | Not more than 0,3% | 0,07 | 0,18 | 0,09 | 0,10 |
| **Quantificatio n** | More than 99,0 | 99,8 | 99,6 | 99,8 | 99,7 |

As can be seen from the table above, the new form of FMP is more stable and the rate of impurity formation in it is much lower.

Better solubility makes it easier and cheaper to manufacture said drug and to avoid the use of potentially toxic solubilizers (solubility enhancers).

The electrostatic sticking reduction demonstrated in the experiment allows to reduce production losses and to optimize the process of industrial production of FMP-based drugs.

## Claims

1. A phenylmethylpyrazolone compound having a crystal form which corresponds to the parameters determined by the Debye-Scherer powder X-ray diffraction method, wherein the parameters of the crystal cells correspond are
| **Parameter\ Unit** | **Result** |
|---|---|
| *a(Å)* | *10.244(6) Å* |
| *b (Å)* | *11.198(5) Å* |
| *c (Å)* | *15.911(9) Å* |
| *β°* | *94.95(3)* ° |
| *VÅ³* | *1821(3) Å³* |

2. The compound according to claim 1, wherein the angles 2Θ for the crystal diffraction patterns correspond to: 4.73°; 5.98°; 9.11°; 9.94°; 11.45°; 14.74°; 15.85°; 17.09°; 21.60°.

3. The compound according to claim 1, wherein the interplanar distances of the crystals correspond to: 8.09 Å; 6.42 Å; 4.58 Å; 3.97 Å; 3.12 Å; 2.79 Å; 2.58 Å; 2.37; 1.99 Å.

4. A process of obtaining the compound having a crystal form according to claims 1-3, said process involving recrystallization of the obtained substance and comprising the following steps: i) dissolving the crystals obtained in the synthesis process in ethanol by heating to 45 ° C under reflux; ii) filtering the resulting solution without cooling through an inert material filter; iii) cooling the solution to 5 ° C to form a precipitate (crystals); iv) washing the crystals obtained with ethyl acetate to remove impurities; v) drying the washed crystals in a rotary evaporator under vacuum.

5. The process of obtaining the compound according to claims 1-4, wherein stage i) is performed in 65% ethanol.

6. The process of obtaining the compound according to claims 1-4, wherein stage ii) is performed by means of a filter made of the inert material selected from the group: fiberglass, steel, polypropylene.

7. The process of obtaining the compound according to claims 1-4, wherein step iv) is carried out at room temperature with ethyl acetate solution.

8. The process of obtaining the compound according to claims 1-4, wherein stage v) is carried out at the temperature of 30 ° C.
